# EUROPEAN PATENT SPECIFICATION

(11) **EP 1 948 101 B1**
(45) Date of publication and mention of the grant of the patent: **21.12.2011**
(21) Application number: 06837097.2
(22) Date of filing: 07.11.2006
(51) Int. Cl.: A61F 2/90

(54) **STENT WITH ORIENTATION-DEPENDENT PROPERTIES**
STENT MIT AUSRICHTUNGSABHÄNGIGEN EIGENSCHAFTEN
STENT DOTE DE PROPRIETES DEPENDANT DE SON ORIENTATION

(30) Priority: 07.11.2005 US 734053 P
(43) Date of publication of application: 30.07.2008
(73) Proprietor: Cook Medical Technologies LLC, Bloomington, IN 47402 (US)
(72) Inventor: BOWE, Jason, S., West Lafayette, IN 47906 (US); OSBORNE, Thomas, A., Bloomington, IN 47401 (US)
(74) Representative: Ellis, Katherine Elizabeth Sleigh
(86) International application number: PCT/US2006/043392
(87) International publication number: WO 2007/053791

(56) References cited:
- EP-A2- 1 236 447
- WO-A-99/25273
- WO-A-2006/032902
- FR-A- 2 785 174
- US-A1- 2005 149 168

## Description

### Technical Field

The present invention generally relates to an implantation device which is curved in a relaxed condition,

### Background of the Invention

Known stents are typically designed with a generally cylindrical structure to allow the stent to be placed in any circumferential orientation without affecting stent design properties. However, some vessels are generally curved, As a result, implantation of a stent within a generally curved vessel results In loading along a particular direction. Conventional stents that are designed to be placed in any circumferential orientation are generally less capable of incurring loading In a particular direction.

Moreover, some vessels with a general curvature have a tendency to undergo severe bending such that their angle of curvature may continuously change. The portion of the suprafemoral artery (SFA) overlying the knee is an example of a curved vessel which undergoes significant changes in its angle of curvature due to bending of the knee, Stents that are typically straight when deployed will be subject to fatigue from the continuous changes in curvature of the SFA and may ultimately fail.

When conventional stents which are straight along their length are deployed within a vessel they exert a continuous radial force on the vessel by attempting to straighten the lumen's natural curvature.

WO 99/25273 discloses an interluminal connector device comprising a plurality of radially expandable annular members having one or more elongate strut members extending therebetween. The device is mountable on or within a delivery catheter while in a radially compact configuration. After the delivery catheter has been inserted into the body, the device is caused to transition to a radially expanded configuration whereby it becomes implanted within the body so as to maintain a desired fluidic connection between adjacent lumens.

EP 1236447 discloses a flexible stent for use inside the lumen of a duct to relieve an obstruction. The stent comprises a flexible section providing flexibility for delivery of the stent.

The stent has structural elements with peaks and valleys that are connected by linkages, where the connection points of the linkages to the structural elements can be such that bending of the stent can be achieved.

US 2005/0149168 discloses a stent comprising a segment having a plurality of interconnected closed serpentine circumferential bands, The stent includes connecting elements that allow bending of the stent so that the stent may be adaptable for deployment within bodily lumens having varying degrees of curvature.

The present invention seeks to provide an improved intraluminal device.

### Summary of the Invention

According to an aspect of the present invention, there is provided an intraluminal device as specified in claim 1.

The preferred embodiments can provide an intraluminal device, such as a stent, which is able to withstand asymmetrical loads incurred from conformance to the curvature of a curved vessel that may later its curvature during a person's movement. The preferred embodiments described below may thus be useful in withstanding asymmetrical loads incurred from conforming to the curvature of a curved vessel and can also solve other problems, as will be apparent from the description above,

Thus the preferred embodiments provide an intraluminal device with a curved configuration that is able to conform to the curvature of the vessel the intraluminal device in which it is implanted.

In a first embodiment, an intraluminal device is provided in accordance with claim 1.

The intraluminal device may Include a stent for deployment within the portion of the suprafemoral artery that overlies the knee.

Said first linkages and said second linkages may comprise flexible portions able to bend when said tubular member is in a generally straight configuration and may be able at least partially to straighten when said tubular member is in said generally curved configuration.

Each of the first linkages may be substantially longitudinally inexpandable and each of the second linkages may be flexible so as to be longitudinally expandable. Each second linkage, when expanded, may have a longitudinal length that is at least 1% greater than the lengths of the first linkages.

The first linkages may extend along a common radial position along the length of the generally tubular member and the second linkages may extend along an opposed radial position along the generally tubular member. This curves the generally tubular member In a single plane. Alternatively, the first linkages may be provided in a plurality of radial positions along at least a portion of the length of the tubular member, In this case, there may be provided respectively positioned second linkages, thereby to give the generally tubular member a curvature in a plurality of planes.

The device may include at least two first linkages of different lengths relative one another; and/or including at least two second linkages of different lengths relative one another.

The first linkages and/or second linkages may have a variable longitudinal dimension, optionally wherein said variable longitudinal dimension is provided by a bend or curve in the linkage.

One or more of the structural elements, the first linkages and the second linkages may be formed from a superelastic alloy, optionally wherein the super elastic alloy is nitinol. The device may also or alternatively include at least one radiopaque marker indicating the radial orientation of the device. The device may also or alternatively include at least one of the following: a stent, a stent-graft, a filter and an occlusion device.

The generally tubular member may adopt a substantially cylindrical configuration when in a compressed state.

When the device is compressed the overall lengths of the portion of the member including the series of first linkages and the portion of the member including the series of second linkages may be substantially the same, so as to give the Intraluminal device an overall shape that is substantially straight.

The structural elements may be aligned with no offset. Optionally, adjacent structural elements may be configured to appear as mirror images of one another.

The present invention also provides for a delivery device including a catheter, an intraluminal device according to any preceding embodiment and a sheath covering the intraluminal device, wherein the intraluminal device is In a compressed configuration on the catheter and substantially straight.

Embodiments of the present invention are described below, by way of example only, with reference to the accompanying drawings, in which:
Figure 1 is a cross-sectional view of a curved stent implanted in a curved blood vessel;
Figure 2 is a partial flat layout view of a curved stent in a flat expanded state with solid fixed length linkages along both a first side and second side of the stent;
Figure 3 is a partial flat layout view of a curved stent in a compressed state;
Figure 4 is a partial flat layout view of a curved stent having flex linkages along both a first side and a second side of the curved stent;
Figure 5 is a partial flat layout view of a curved stent having valley-to-valley fixed length linkages extending along a first side of the curved stent and valley-to-valley flexed linkages in an unexpanded state and extending along a second side of the curved stent;
Figure 6 is a partial flat layout view of the curved stent of Figure 5 in an expanded state;
Figures 7a-f are various stent geometries having various bends along its length in accordance with principles of the embodiments; and
Figure 8 is a cross-sectional view of a stent collapsed onto a delivery catheter and advanced to an implantation site within a curved blood vessel.

It is to be understood that the drawings are not to scale and in certain instances details, which are not necessary for an understanding of the embodiments, have been omitted, such as conventional details of fabrication and assembly.

### Detailed Description

The invention as generally described herein is directed to intraluminal devices, such as stents, stent grafts, filters, and the like. However, for purposes of clarity, the drawings and description are directed to various embodiments of stent. However, it should be understood that the teachings herein apply to other intraluminal devices.

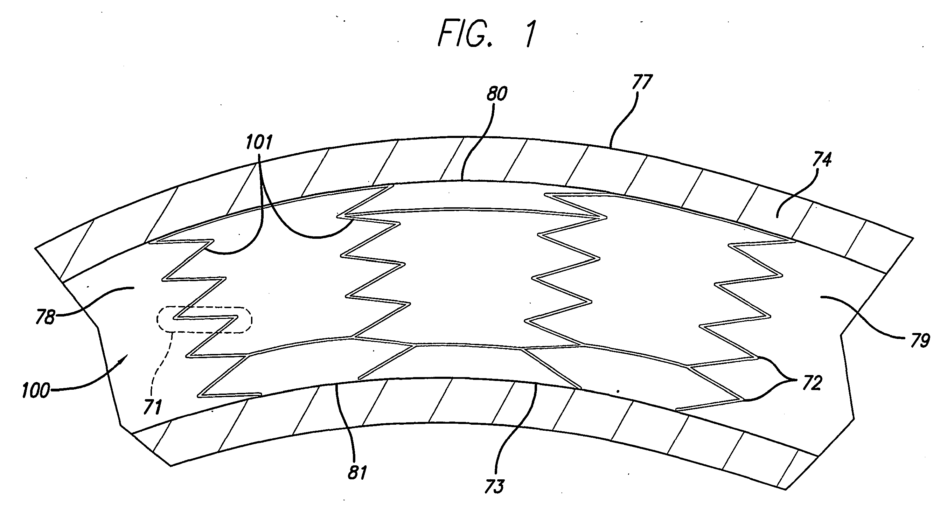

Figure 1 is a cross-sectional view showing an embodiment of curved stent 100 implanted within a curved lumen of a blood vessel 77. Although Figure 1 shows the stent 100 implanted into a blood vessel 77, the stent 100 may be implanted into any vessel or organ having a curved configuration. The stent 100 has an outer radius of curvature 74 and an inner radius of curvature 73. The curvature of the stent 100 is configured to correspond to the curvature of the blood vessel 77. The stent 100 is a tubular structure which forms a generally curved shape in its relaxed condition. The stent 100 comprises structural elements 101 having a zig-zag configuration. The structural elements 101 extend from a first end 78 to a second end 79 and from a first side 81 to a second side 80.

Although Figure 1 shows zig-zag structural elements, any shaped structural element would be apparent to one of ordinary skill in the art and be within the scope of the teachings herein. The structural elements extending along the second side 80 form the outer radius of curvature 74 and the structural elements extending along the first side 81 form the inner radius of curvature 73.

The second end 79 of the stent 100 may be provided with circular radiopaque markers 72, while the first end 78 of the stent 100 may be provided with radiopaque orientation markers, such as J-shaped markers 71. J-shaped markers 71 provide unique directional indicia to assist a physician in recognizing the orientation of the stent 100 when viewed in a two-dimensional screen such as a fluoroscope. The J-shaped markers 71 are visually indicative of a radial orientation of the stent 100. The makers 71 indicate the orientation of the stent 100 prior to and after its deployment, thereby allowing the physician to position the stent 100 rotationally such that its curvature or bend corresponds to the curvature or bend of the blood vessel 77.

In another embodiment, the radiopaque marker may comprise a single elongated marker on an end of a side of the stent 100. The elongated marker may be aligned with the curvature of the vessel 77. In another embodiment, multiple markers may extend along one side of the stent 100 and may be configured to align with the curvature of the vessel 77. The markers may be positioned at the apices of the struts of the stent 100. Such positioning of the multiple markers may be suitable for determining the orientation of the stent 100 within a vessel having compound or multiple curves. The single elongated and multiple markers are visually indicative of the radial orientation of the stent 100. The markers can be created with current marker riveting techniques as known to those skilled in the art.

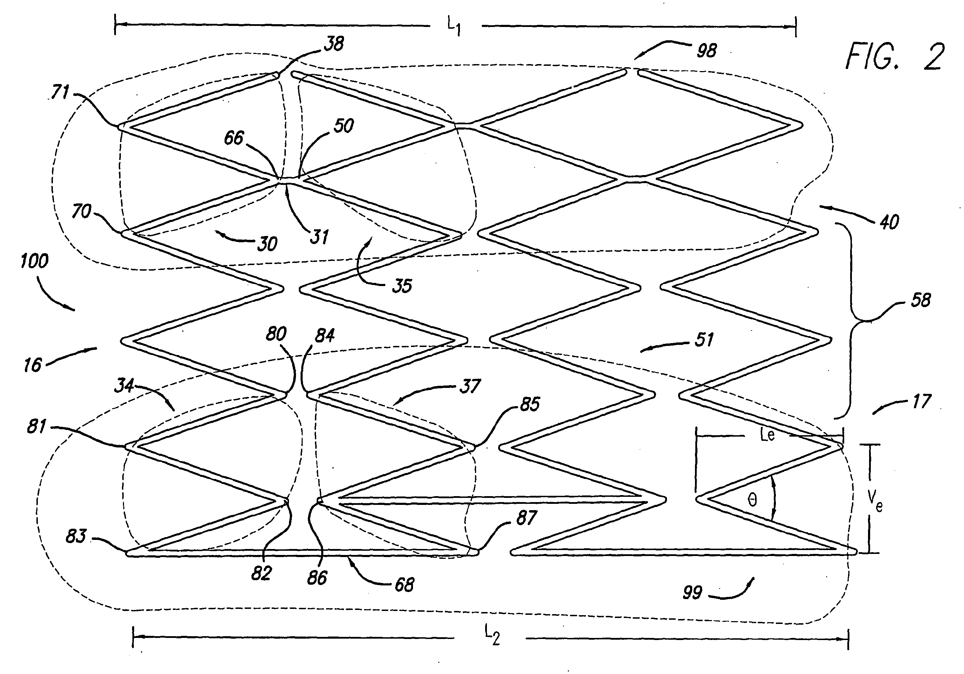

Figure 2 shows an embodiment of stent 100 which for illustration purposes has been unrolled circumferentially into a flat configuration. Only a portion of the unrolled stent 100 is shown in Figure 2. The stent 100 includes a series of structural elements which extend from a first end 16 to a second end 17 and from a first side 98 to a second side 99.

Structural element 30 (shown within the deleted outline) has three members arranged in a zig-zag "Z" shape. Structural element 30 comprises a total of four apices, specifically, peaks 38 and 66 and valleys 70 and 71. Peak 66 of structural element 30 is connected to peak 50 of the adjacent structural element 35 to form a peak-to-peak link 31.

Structural element 35, which is similar to structural element 30, contains two peaks and two valleys. Structural elements 35 and 30 may be aligned with no offset such that the valleys 70 and 71 of structural element 30 are not nested within the peaks of structural element 35 to form a packed configuration. Structural element 35 may be configured to appear as a mirror image of structural element 30, as shown in Figure 2.

Still referring to Figure 2, a series of elements 30 and 35, connected by peak-to-peak links, such as the peak-to-peak link 31, extend from the first end 16 to the second end 17 to create a first region or pattern 40 extending along the first side 98 of the stent 100. The first side 98 has a longitudinal length of L₁.

Structural element 34 has three members arranged in a zig-zag shape. Structural element 34 comprises a total of four apices, specifically peaks 80 and 82 and valleys 81 and 83. Structural element 34 is identical to structural element 30. Valley 83 of structural element 34 is connected to valley 87 of structural element 37 by a valley-to-valley linkage 68.

Structural element 37 is similarly oriented to structural element 35 and contains two peaks 84, 86 and two valleys 85, 87. Structural elements 37 and 34 are aligned with substantially no offset. Structural element 37 may be configured to appear as a mirror image of structural element 34, as shown in Figure 2.

Still referring to Figure 2, a series of valley-to-valley links, such as the valley-to-valley link 68, extend from the first end 16 to the second end 17 within a second region or pattern 51 extending along the second side 99 of the stent 100. The second side 99 has an overall longitudinal length of L₂.

The overall longitudinal length L₂ of side 99 is greater than the overall longitudinal length L₁ of side 98 when the stent is in its relaxed condition, as a result of which the stent 100 has a tendency to form a curved configuration when it is rolled so that its edges 98 and 99 are connected together or at least adjacent one another. The series of valley-to-valley linkages that create the regions or patterns 51 form the outer radius of curvature 74 (Figure 1), while the series of peak-to-peak linkages that create the regions or patterns 40 form the inner radius of curvature 73 (Figure 1).

Referring again to Figure 2, z-shaped side elements 58 are the structural elements that are disposed between patterns 40 and 51. The side elements 58 may possess linkages of a length that allows adjacent ones of the side elements 58 to have a separation distance that is intermediate that of adjacent structural elements 30 connected with peak-to-peak linkages and adjacent structural elements 34 connected with valley-to-valley linkages. Alternatively, the side elements 58 may not possess any linkages, as shown in Figure 2. The absence of linkages within this region allows unidirectional bending of the stent 100 to occur, thereby allowing the desired curvature of the stent 100 to form when it radially expands.

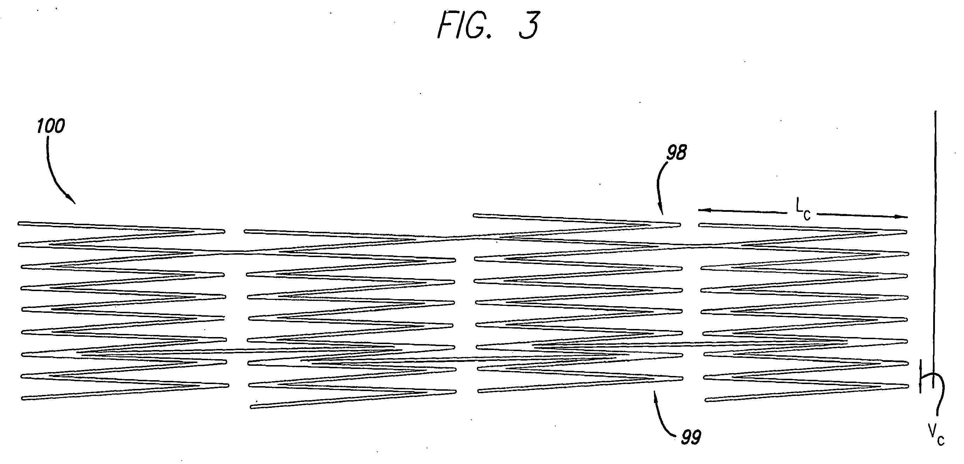

Figure 3 illustrates the stent 100 unrolled, that is in a flat configuration but with its sides pushed towards one another (c.f. Figure 2), to illustrate the features of the stent when it is in its tubular compressed form, for example in its configuration for delivery purposes. The stent 100 is typically in the compressed state when mounted onto a delivery catheter system prior to deployment at a target site. As Figure 3 shows, the first side 98 and second side 99 of the stent are preferably substantially the same length when the stent 100 is radially compressed. Therefore, the overall shape of the stent 100 may be substantially straight before implantation. Thus, the linkages do not prevent the stent 100 from attaining a linear shape when in its compressed state, rather, they assist in straightening of the stent 100.

It is envisaged that in some embodiments the linkages 31 and 68, shown in Figure 2, may be arranged to bend to allow the stent 100 to compress and have a linear shape suitable for advancement within a delivery catheter, which will be discussed below.

To explain, in this embodiment, when in the compressed state the distance between adjacent apices of each of the structural elements, L_{c} (Figure 3), in the longitudinal direction of the stent is greater than the distance in the longitudinal direction of the stent between adjacent apices when the stent is in the expanded state, Lₑ (Figure 2). Additionally, the radial distance between apices of each of the structural elements in the compressed state, V_{c} (Figure 3), is less than the radial distance between apices in the expanded state, Vₑ (Figure 2). Thus, when the stent is radially compressed, the arrangement and lengths of the linkages and strut members is such as to cause the stent 100 to adopt, in the preferred embodiment, a substantially straight or cylindrical configuration.

For the embodiment depicted in Figure 2, during expansion the angular separation between strut members of a Z-shaped structural element (i.e., the included angle of the valleys), θ, increases relative to the angular separation between strut members in the compressed state (Figure 3). As a result, the separation between adjacent Z-shaped structural elements with valley-to-valley linkages (e.g., structural elements 34 and 37) may correspondingly increase. The distance between adjacent Z-shaped structural elements with peak-to-peak linkages (e.g., structural elements 30 and 35) may remain the same. This difference in longitudinal length associated with the regions or patterns 40 and 51 in which peak-to-peak linkages and valley-to-valley linkages are respectively provided causes the stent 100 to attain a curved configuration. This curved configuration allows the stent 100 to have desired unidirectional properties, that is allow the stent 100 to withstand asymmetrical loading which can potentially cause fatigue and eventual failure of the stent 100, as is commonly incurred with prior art stents which are filled in curved vessels.

Figure 2 shows the peak-to-peak linkages 31 and the valley-to-valley linkages 68 apparently adjacent the opposing sides 98, 99 of the stent when unrolled as shown. However, the actual radial positioning of these linkages 31, 68 will be dependent upon the curvature desired for the stent 100. For the example of Figure 1, in which the stent is to have a simple curved shape, the links 31 and 68 are preferably radially opposite one another, that is substantially at 180° to one another.

It is to be appreciated that a complex curvature of the stent 100 can be achieved by locating the links 31 and 68 differently between sections 30, 35, 34, 37 and so on. For example, valley-to-valley links 68 could be arranged in different radial positions along the length of the stent 100, with peak-to-peak linkages 31 arranged in corresponding manner, to give the stent 100 both curvature and twist when in its expanded configuration.

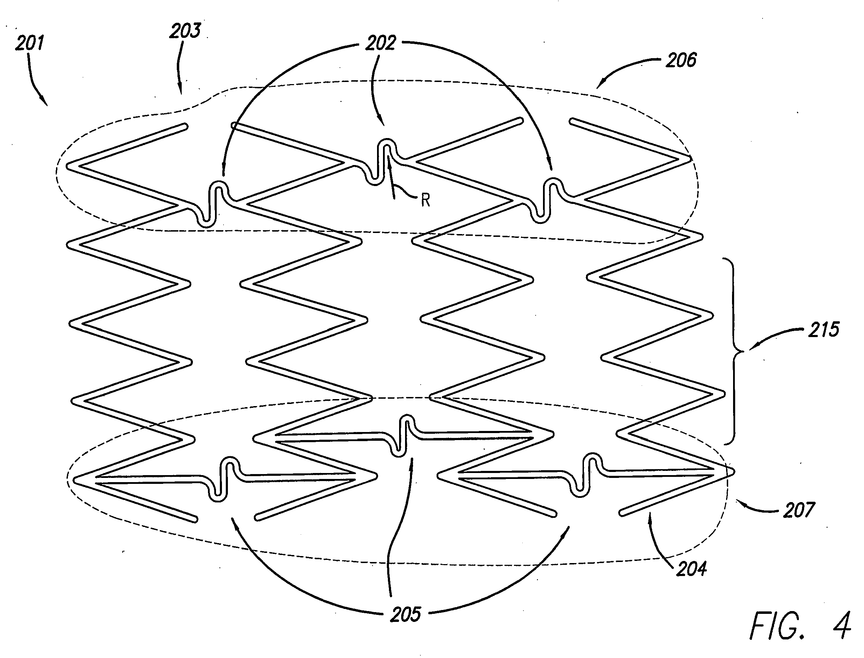

Figure 4 is a partial flat layout view of an unrolled section for illustration of a stent 201 provided with flex linkages. The stent 201 is shown in an expanded state which it attains when deployed into a vessel. Figure 4 illustrates peak-to-peak flex linkages 202 extending along a first side 203 of the stent 201 to form a first pattern 206. Valley-to-valley flex linkages 205 extend along a second side 204 of the stent 201 to form a second pattern 207. Side structural elements 215 are disposed between the first pattern 206 and the second pattern 207. Preferably, side structural elements 215 are not connected by flex linkages in order to facilitate unidirectional bending at the location of the side structural elements 215.

The arrangement and lengths of the linkages 202, 205 is preferably such that the stent 201, when tubular and compressed, is substantially straight. Thus, when it is expanded from this compressed form, the series of peak-to-peak flex linkages 202 along the first side 203 and a series of valley-to-valley flex linkages 205 along the second side 204 causes the longitudinal length of the stent 201 to be greater along the second side 204. This difference in length causes the stent 100 to have a tendency to form a curved configuration when it radially expands within a vessel (Figure 1). The series of valley-to-valley flex linkages may form the outer radius of curvature 74 (Figure 1). The series of peak-to-peak flex linkages may form the inner radius of curvature 73 (Figure 1).

The comments made above in connection with the embodiment of Figure 2 with respect to the positioning of the peak-to-peak and valley-to-valley links apply equally to this embodiment.

Still referring to Figure 4, R is the radius of the flex linkages 202 and 205. R may be varied to achieve the degree of flexibility desired in a curved vessel. For example, R may be increased for curved vessels having severe bending. A large radius R facilitates asymmetrical loading. A large radius is also suitable for lumens where there is not only a curvature, but the curvature changes due to body movement, as with the SFA portion located over the knee. R may be decreased for curved vessels where the curvature is relatively constant. A variety of factors may be considered in determining a suitable radius, including the vessel the stent 201 is to be implanted into, the severity of bending of the vessel, and the frequency, if any, at which the curvature of the vessel bends and changes. Determining a suitable radius of flexed linkages 202 and 205 for a particular curved vessel on the basis of such factors will be apparent to one of ordinary skill in the art having regard to the teachings herein.

Since the flex linkages 202 and 205 provide the stent 201 with flexibility, the stent 201 may accommodate the loads incurred during severe bending of a curved body artery, such as the SFA. The section of the SFA over the knee is an example of a blood vessel having a bend that can continuously change due to the bending of the knee. The stent 201 may be able to adapt to continuous changing angles of curvature of the SFA without fatiguing. Known stents that cannot accommodate such asymmetrical loading may be subject to fatigue and may ultimately fail.

The radius of curvature of the flexed links 202 may also be different than the radius of curvature for flexed links 205. In an embodiment, the radius of curvature of the flexed links 205 is greater than that of flexed links 202.

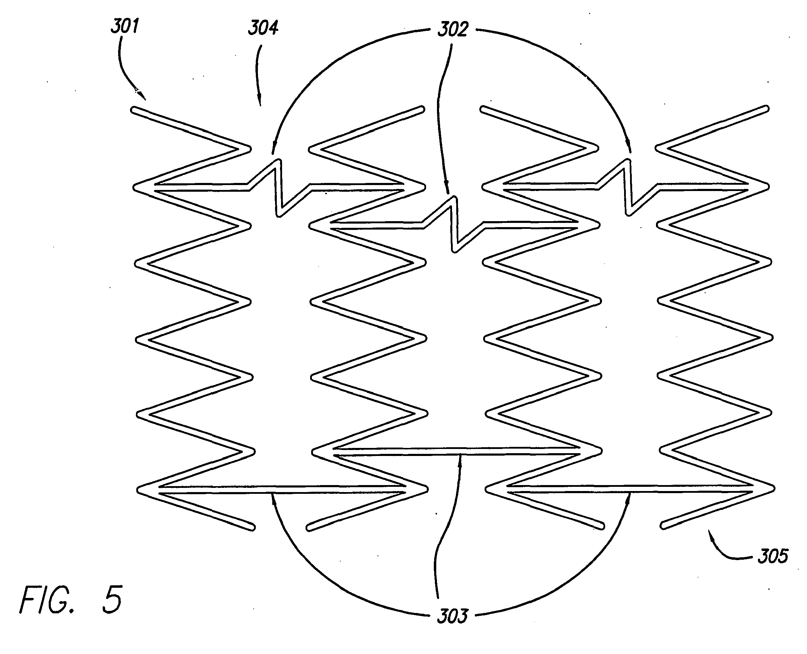

Figure 5 shows a stent not part of the invention 301 with a combination of flex linkages and fixed length linkages. Figure 5 illustrates an intermediate snapshot of the stent 301 undergoing expansion. Each of the z-shaped structural elements is radially expanded. However, the flex linkages 302 are not yet longitudinally expanded, shown still in their collapsed configuration. Flex linkages 302 extend along a second side 304 of the stent 301 and fixed solid linkages 303 extend along a first side 305 of the stent 301. Figure 5 shows that both the flex linkages 302 and the fixed solid linkages 303 connect valleys of adjacent structural elements of the stent 301. Fixed solid linkages 303 may remain constant in length when in both the expanded state and in the collapsed state.

The flex linkages 302 and fixed solid linkages 303 may also be peak-to-peak linkages in which adjacent structural members of the stent 301 are connected to each other at their respective peak points. A difference in overall length may occur when the flex linkages 302 expand from their collapsed state. The pattern created by the series of flex linkages 302 may extend by a greater longitudinal length than the pattern created by the series of fixed solid linkages 303. This causes the stent 301 to attain a curved configuration with unidirectional, properties. The effect of such a curved configuration is that the stent can conform to the shape of a curved vessel.

The comments made above in connection with the embodiment of Figure 2 with respect to the positioning of the various interconnection links apply equally to this embodiment.

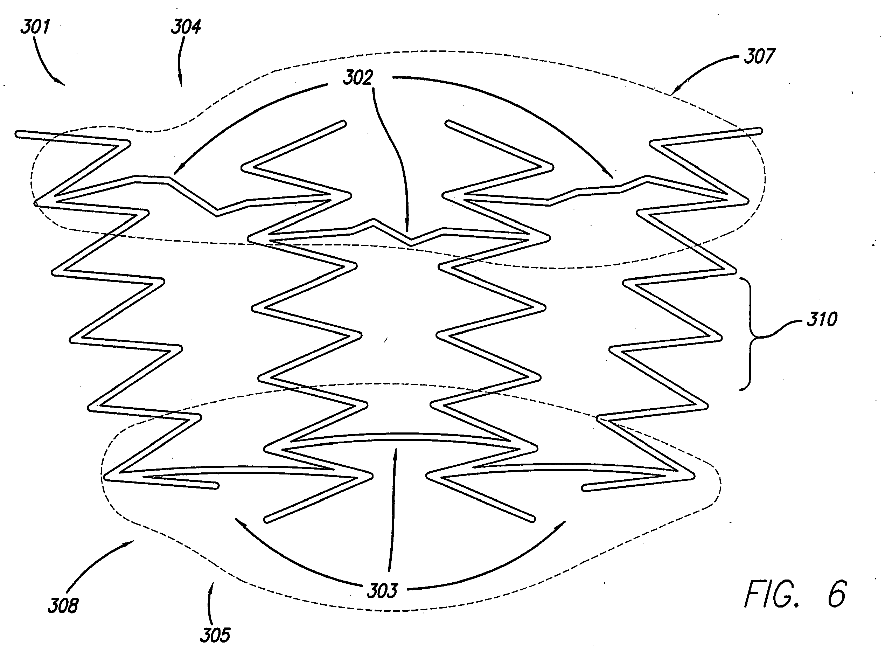

Figure 6 shows the unrolled stent 301 with the flex linkages 302 as described in Figure 5 in a longitudinally expanded condition. This may be the configuration the stent 301 attains when deployed and expanded in an implanted curved vessel. The flex linkages 302 partially straighten in the longitudinal direction when expanded, thereby providing for the stent to curve. The extent to which the flex linkages longitudinally straighten is dependent upon a number of factors, including the extent of bend or curvature of the vessel and the type of vessel into which the stent 301 is implanted. The fixed linkages 303 may remain constant in length. The result is that portion or pattern 307 extends a greater longitudinal length than portion or pattern 308, thereby causing the stent to take a curved configuration when radially expanded. Flex linkages 302 and portion or pattern 307 form the outer radius of curvature 74, shown in Figure 1. Fixed linkages 303 and portion or pattern 308 form the inner radius of curvature 73, shown in Figure 1.

The expansion process of the stent 301 provides for radial expansion of each of the z-shaped structural elements, as shown in Figure 5, and longitudinal expansion of each of the flex linkages 302, as shown in Figure 6. Both expansions may occur simultaneously upon deployment into a vessel.

Intermediate linkages (not shown) may be provided between the portions or patterns 307 and 308. The intermediate linkages may be peak-to-peak linkages in which adjacent structural elements 310 may be connected at their respective peak points. Alternatively, the intermediate linkages may be valley-to-valley linkages in which adjacent structural elements 310 may be connected at their respective valley points. Such intermediate linkages may have a length proportionally less than the linkages which form the outer radius of curvature 74 and a length proportionally greater than the linkages which form the inner radius of curvature 73.

The arrangement of linkages, as viewed from one of the ends of the stent when in its tubular form, is now discussed. Referring to Figure 6, the flex linkages 302 and corresponding portion or pattern 307 that form the outer radius of curvature 74 (Figure 1) may be located at the 12 o'clock position. The fixed linkages 303 and corresponding portion or pattern 308 that form the inner radius of curvature 73 (Figure 1) may be located at the 6 o'clock position. Similarly, in the embodiment of Figure 2, the valley-to-valley fixed linkages forming portion or pattern 51 may be located at the 12 o'clock position while the peak-to-peak fixed linkages forming portion or pattern 40 may be located at the 6 o'clock position. In the embodiment of Figure 4, the valley-to-valley flex linkages 205 forming portion or pattern 207 may be located at the 12 o'clock position while the peak-to-peak flex linkages forming portion or pattern 206 may be located at the 6 o'clock position. Although not shown, intermediate linkages may be arranged that have a length proportionally between the length of linkages associated with the outer radius of curvature 74 and inner radius of curvature 73.

As explained above in connection with Figure 2, these portions of the various linkages are only an example. The linkages can be located along the length and radial positions of the stent to produce substantially any desired curvature. In one example, the stent could be made to have a natural curvature in two planes, achieved by locating peak-to-peak links and valley-to-valley links, for example, at the 6 and 12 o'clock positions in a first part of the stent and at different positions, such as the 3 and 9 o'clock positions, along a second part of the stent. In another example, the stent could have a natural spiralling configuration.

Similarly, the length of the various linkages could be varied to vary the amount of curvature of the stent or stent part. Again, the stent could be provided with an even radius of curvature throughout its length as it could be provided with different radii of curvature.

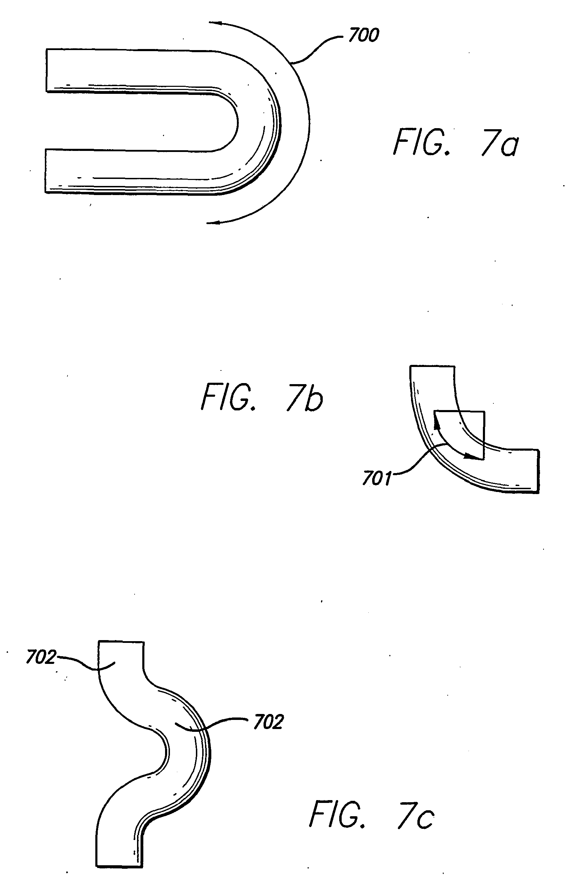

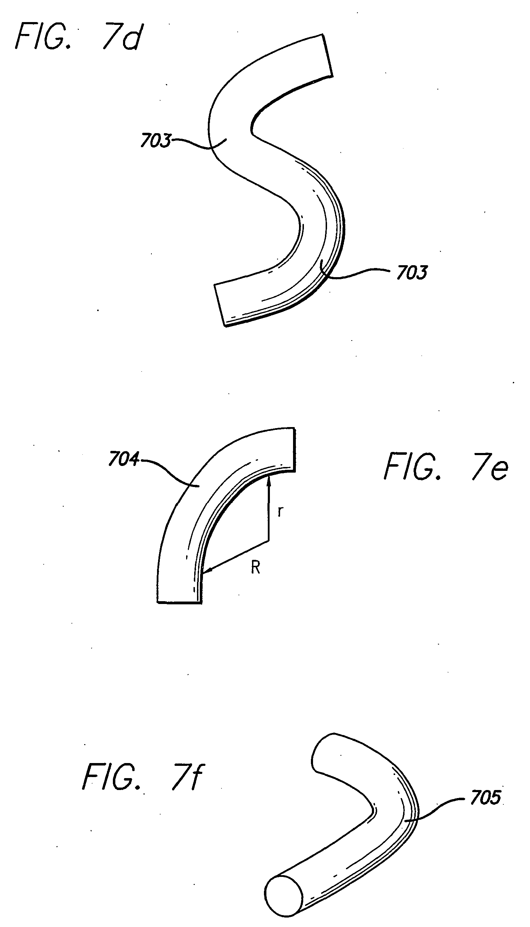

The structural features of the embodiments described above allow stents to be designed with various bends and curves along the length by selectively placing linkages along the circumference of a stent. For example, Figure 7a shows a stent with a 180° bend 700 along its length. In Figure 7b, a stent shows a 90° bend 701 along its length. Figure 7c shows a stent with multiple bends 702 along its length. Figure 7d also shows a stent with multiple bends 703. Figure 7e shows a stent having a compound curve 704 in which the compound curve 704 has at least two radii of curvature, r and R. Figure 7f shows a stent having a bend 705 that extends into three dimensions. In particular the bend occurs along plane x-y, along plane x-z, and along plane y-z. Any and all combinations of the foregoing and other bends and curves known in the art are also contemplated. Selective positioning of the linkages to achieve the bends illustrated in Figures 7a-f will be from the above teachings.

The stent of the various described embodiments may be made from a superelastic alloy. Preferably, the superelastic alloy is a nickel-titanium alloy, such as nitinol. Nitinol may undergo a substantially reversible phase transformation that allows it to "remember" and return to a previous shape or configuration. The phase transformation may occur between an austenitic phase and a martensitic phase. The phase transformation may occur by cooling the stent below its phase transformation temperature (shape memory effect). Additionally and more preferably, the phase transformation may occur by applying stress to the stent, thereby stress-inducing martensite in what is known as the superelastic effect. In one example utilizing the superelastic effect, stress may applied to nitinol having an initial shape in the austenitic phase to cause a transformation to the martensitic phase without a change in temperature. A return transformation to the austenitic phase may be achieved by removing the applied stress. In general, superelastic alloys are elastic over a wider range than conventional elastic materials such as stainless steel. For example, nitinol can have an elastic range of up to about 8%.

The embodiments as described herein preferably utilize the superelastic properties of nickel-titanium alloys, By virtue of the superelastic properties of such alloys, the stent tends to spring back naturally to a larger diameter when a restraining stress is removed. Accordingly, the stress introduced into the stent may be released by withdrawing the restraining sheath in a proximal direction away from the stent, whereupon the stent expands to its original, curved shape by transforming back to the austenitic phase.

Preferably, the stent 100 (Figure 1) may be unitary, meaning that it is constructed from a single piece of material. For example, the stent 100 may be cut to length from stock nitinol tubing. The tubing may then be laser cut to form the desired pattern with the desired linkages. A unitary stent construction avoids process variances of other mechanically linked stents.

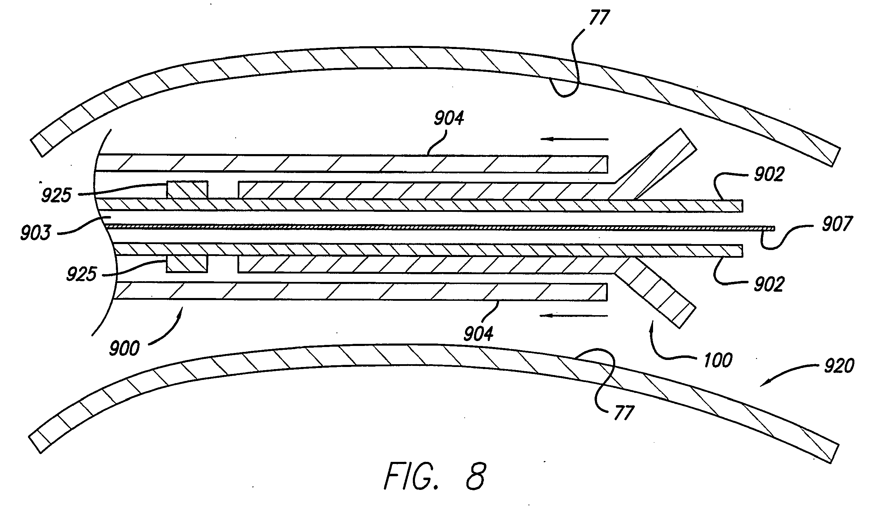

Figure 8 is a cross-sectional view showing the stent 100 in a partially compressed delivery configuration within the delivery catheter 900. The delivery catheter 900 comprises an inner sheath 902 which may have a guide wire lumen 903 designed to receive a guide wire 907. Delivery catheter 900 also includes an outer sheath 904 which has a lumen sized to fit around the inner sheath 902. The outer sheath 904 is retractable and extends over inner sheath 902 and stent 100 in order to maintain the stent 100 in the collapsed delivery configuration. The outer sheath 904 maintains a compressive force over the stent 100, as a result of which the compressed stent 100 maintains a small profile during delivery to the implantation site 920. The superelastic characteristics of the stent 900 generally allow it to be compressed as shown. The restraining sheath 904 and inner sheath 902 enable the stent 100 to straighten while it is in the delivery configuration, thereby facilitating delivery of the stent 100 to the implantation site 920. Compression of the stent 100 allows the stent 100 to remain in place on the delivery catheter 900 during advancement of the delivery catheter 900. Other means for securing the stent 100 onto the delivery catheter 900 may also be used, such as collars or ridges.

A method of using the stent 100 within a curved blood vessel 77 is now described. Generally speaking the delivery catheter 900 with the stent 100 disposed thereon in the collapsed configuration (that is with the outer sheath 904 completely covering the stent 100) is advanced over the guide wire 907 to an implantation site 920 within the curved blood vessel 77.

The J-shaped gold markers 71 located on the first end 78 (Figure 1) of the stent 100 and the normal circular markers 72 located on the second end 79 of the stent facilitate visualization of the alignment and orientation of the stent 100 within the implantation site during and after deployment. The markers 71 and 72 allow the stent 100 to be deployed in a specific orientation in order for the curvature or bend of the stent 100 to conform to the curvature or bend of the blood vessel 77. Longitudinal positioning of the stent 100 may be accomplished by monitoring the markers 71 and 72 on an x-ray screen and orienting the ends of the stent 100, as shown by the J-shaped gold markers 71 and the normal circular markers 72, within the implantation site 920. The implantation site 920 may be fluoroscopically mapped as known by one of ordinary skill in the art.

Radial positioning of the stent 100 may be required in order to align rotationally the curvature of the stent 100 with the curvature of the blood vessel 77. The bend in the stent 100 preferably follows the bend in the vessel 77 upon deployment of the stent 100. It is therefore useful for the physician to know beforehand the location and direction of the bend in the stent 100 prior to deployment such that the outer radius of curvature 74 (Figure 1) and inner radius of curvature 73 (Figure 1) of the stent 100 may correspond with the bend of the vessel 77. The J-shaped gold markers 71 provide unique directional indicia to assist the physician in such recognition of the desired curved orientation of the stent 100 when viewed as a two-dimensional screen such as a x-ray screen.

The J-shaped gold markers 71 may be a solid J-shape. Alternatively, the J-shape gold markers 71 may be formed from discrete markers oriented orthogonally to create the J-shape. Other variations of J-shaped markers are contemplated and are within the scope of the teachings herein.

As an alternative to a stent 100 with normal circular radiopaque markers 72 on its second end 79 and radiopaque orientation markers, such as J-shaped markers 71, on its first end 78, the radiopaque markers may be small rivets composed of a radiopaque material such as gold, tantalum, platinum, or palladium. The markers may be arranged so that there are two markers, one on each end of the stent 100 positioned on the outer radius of curvature 74. Three or more markers may be arranged along the length of the stent 100 on the inner radius of curvature 73. The physician may then orient the stent 100 rotationally using fluoroscopic guidance before deploying the stent 100. The markers may be placed in small eyelets in the stent 100 structure in a manner similar to conventional stents where the markers are used to identify the ends of the stent 100. This arrangement would not only provide rotational information, but also location information regarding the ends of the stent 100.

Once the stent 100 has been radially and longitudinally oriented with respect to the implantation site 920 within the blood vessel 77, the outer sheath 904 of the delivery catheter 900 is gradually retracted, as indicated by the arrows in Figure 8. A shoulder 925 or other longitudinal restraint known in the art prevents the stent from moving backwards into the delivery catheter as the outer sheath 904 is withdrawn.

The relative movement between the inner sheath 902 and outer sheath 904 may be accomplished by manipulation of control handles (not shown) located at the proximal end of the delivery catheter 900. Regardless of what method for retracting the outer sheath 904 is used, when the distal end of outer sheath 904 is retracted to release the stent 100, as shown in Figure 8, the stent 100 self-expands to its naturally curved configuration, as shown in Figure 1. The curvature of the stent 100 conforms to the curvature of the implantation site 920 within the blood vessel 77. After the stent 100 is implanted and contacts the implantation site 920, the delivery catheter 900 and guide wire 907 are withdrawn from the patient's vasculature, completing the procedure.

Although the above-described embodiments relate generally to self-expanding stents, the teachings herein apply equally to stent structures which are expandable by an expansion device, such as a balloon, bulbous expansion member or other known or suitable device. Such a stent would still provide a natural, unbiased, shape of the types disclosed herein. Furthermore, although the embodiments described above relate to stents, the teachings herein are applicable to many other types of implants including, for example, stent-grafts, filters, occlusion devices and so on. Furthermore, in some instances it may be desired to cover only a part of an implant in the manner taught herein. Examples include an implant which includes a stent graft coupled to an elephant trunk section, a branched implant and so on.

## Claims

1. An intraluminal device (100), including:
a series of structural elements (30, 35, 34, 37) extending in a generally longitudinal direction to form a generally tubular member and comprising sections arranged to provide a series of peaks (38, 50, 66, 80, 82, 84, 86) and valleys (70, 71, 83, 85, 87), said structural elements being interconnected to one another by at least one series of first linkages (31, 202) and at least one series of second linkages (68, 205), wherein said first linkages are shorter in length than said second linkages, said first and said second linkages being disposed radially and longitudinally along said generally tubular member,
the device being **characterised in that** a first series of structural elements (30, 35) is contained in a first region (40) extending along a first side (98) of the member with peaks (66) of one structural element (30) being connected to peaks (50) of an adjacent structural element (35) by first linkages, **in that** a second series of structural elements (34, 37) is contained in a second region (51) extending along a second side (99) of the member with valleys (83) of one structural element (34) being connected to valleys (87) of an adjacent structural element (37) by second linkages, and **In that** the arrangement and lengths of the linkages and strut members is such that, when radially compressed, the generally tubular member is caused to adopt a substantially straight shape and, when radially expanded and in a relaxed condition, the first region (40) has an overall length (L₁) shorter than the overall length (L₂) of the second region (51) so as to give said generally tubular member a curved shape.

2. An intraluminal device (100) according to claim 1, wherein the intraluminal device includes a stent for deployment within the portion of the suprafemoral artery that overlies the knee.

3. An intraluminal device (100) according to any preceding claim, wherein said first linkages (202) and said second linkages (205) comprise flexible portions able to bend when said tubular member is in a generally straight configuration and are able at least partially to straighten when said tubular member is in said generally curved configuration.

4. An intraluminal device (100) according to any preceding claim, wherein each of the first linkages (303) is substantially longitudinally inexpandable and each of the second linkages (302) is flexible so as to be longitudinally expandable; wherein each second linkage, when expanded, has a longitudinal length that is at least 1% greater than the lengths of the first linkages.

5. An intraluminal device (100) according to any preceding claim, wherein the first linkages (31) extend along a common radial position along the length of the generally tubular member and the second linkages (68) extend along an opposed radial position along the generally tubular member; thereby curving the generally tubular member in a single plane.

6. An intraluminal device according to any one of claims 1 to 5, wherein first linkages (31) are provided in a plurality of radial positions along at least a portion of the length of the tubular member, there being provided respectively positioned second linkages (68), thereby to give the generally tubular member a curvature in a plurality of planes.

7. An intraluminal device (100) according to any preceding claim, including at least two first linkages (31) of different lengths relative one another; and/or including at least two second linkages (68) of different lengths relative one another,

8. An Intraluminal device (100) according to any preceding claim, wherein the first linkages (31) and/or second linkages (68) have a variable longitudinal dimension, optionally wherein said variable longitudinal dimension is provided by a bend or curve in the linkage.

9. An intraluminal device (100) according to any preceding claim,
wherein one or more of the structural elements (30, 35), the first linkages (31) and the second linkages (68) are formed from a superelastic alloy, optionally wherein the super elastic alloy is nitinol; and/or
wherein the device includes at least one radiopaque marker (71, 72) indicating the radial orientation of the device; and/or
wherein the device includes at least one of the following: a stent, a stent-graft, a filter and an occlusion device.

10. An intraluminal device (100) according to any preceding claim, wherein the generally tubular member adopts a substantially cylindrical configuration when in a compressed state.

11. An intraluminal device (100) according to any preceding claim, wherein when the device is compressed the overall lengths of the portion of the member including the series of first linkages (31) and the portion of the member including the series of second linkages (68) are substantially the same, so as to give the intraluminal device an overall shape that is substantially straight.

12. An intraluminal device (100) according to any preceding claim, wherein the structural elements (30, 35) are aligned with no offset, optionally wherein adjacent structural elements (30, 35) are configured to appear as mirror images of one another.

13. A delivery device including a catheter (900), an Intraluminal device (100) according to any preceding claim and a sheath (904) covering the intraluminal device; wherein the intraluminal device is in a compressed configuration on the catheter and substantially straight.

## Patentansprüche

1. Intraluminale Vorrichtung (100), die Folgendes umfasst: eine Reihe von strukturellen Elementen (30, 35, 34, 37), die sich in einer allgemeinen Längsrichtung zur Bildung eines allgemein röhrenförmigen Glieds erstrecken und Abschnitte umfassen, die so angeordnet sind, dass sie eine Reihe von Spitzen (38, 50, 66, 80, 82, 84, 86) und Tälern (70, 71, 83, 85, 87) bieten, wobei die strukturellen Elemente durch zumindest eine Reihe von ersten Verknüpfungen (31, 202) und zumindest eine Reihe von zweiten Verknüpfungen (68, 205) miteinander verbunden sind, worin die ersten Verknüpfungen kürzer sind als die zweiten Verknüpfungen, wobei die ersten und zweiten Verknüpfungen radial und längs auf dem allgemein röhrenförmigen Glied angeordnet sind, wobei die Vorrichtung **dadurch gekennzeichnet ist, dass** eine erste Reihe von strukturellen Elementen (30, 35) in einer ersten Region (40), die sich entlang einer ersten Seite (98) des Glieds erstreckt, enthalten sind, wobei Spitzen (66) eines strukturellen Elements (30) durch erste Verknüpfungen mit Spitzen (50) eines benachbarten strukturellen Elements (35) verbunden sind, dass eine zweite Reihe von strukturellen Elementen (34, 37) in einer zweiten Region (51), die sich entlang einer zweiten Seite (99) des Glieds erstreckt, enthalten sind, wobei Täler (83) eines strukturellen Elements (34) über zweite Verknüpfungen mit Tälern (87) eines benachbarten strukturellen Elements (37) verbunden sind, und dass die Verknüpfungen und Strebenglieder so angeordnet sind und solche Längen aufweisen, dass das allgemein röhrenförmige Glied bei radialer Kompression eine im Wesentlichen gerade Gestalt annimmt und die erste Region (40) bei radialer Expansion und in einem entspannten Zustand eine Gesamtlänge (L₁) aufweist, die kürzer ist als die Gesamtlänge (L₂) der zweiten Region (51), um dem allgemeinen röhrenförmigen Glied eine gekrümmte Gestalt zu verleihen.

2. Intraluminale Vorrichtung (100) nach Anspruch 1, worin die intraluminale Vorrichtung einen Stent zur Ablage in dem Abschnitt der suprafemoralen Arterie, die über dem Knie liegt, aufweist.

3. Intraluminale Vorrichtung (100) nach einem der vorhergehenden Ansprüche, worin die ersten Verknüpfungen (202) und die zweiten Verknüpfungen (205) flexible Abschnitte umfassen, die sich biegen können, wenn sich das röhrenförmige Glied in einer allgemein geraden Konfiguration befindet, und die sich zumindest teilweise gerade strecken können, wenn sich das röhrenförmige Glied in der allgemein gekrümmten Konfiguration befindet.

4. Intraluminale Vorrichtung (100) nach einem der vorhergehenden Ansprüche, worin jede der ersten Verknüpfungen (303) in Längsrichtung im Wesentlichen nicht expandierbar ist und wobei jede der zweiten Verknüpfungen (302) flexibel ist, um in Längsrichtung expandierbar zu sein; worin jede zweite Verknüpfung nach der Expansion eine Länge in Längsrichtung aufweist, die zumindest 1% größer ist als die Längen der ersten Verknüpfungen.

5. Intraluminale Vorrichtung (100) nach einem der vorhergehenden Ansprüche, worin sich die ersten Verknüpfungen (31) entlang einer gemeinsamen radialen Position über die Länge des allgemein röhrenförmigen Glieds erstrecken und sich die zweiten Verknüpfungen (68) entlang einer gegenüberliegenden radialen Position über das allgemein röhrenförmige Glied erstrecken; wodurch das allgemein röhrenförmige Glied in einer einzelnen Ebene gekrümmt wird.

6. Intraluminale Vorrichtung nach einem der Ansprüche 1 bis 5, worin erste Verknüpfungen (31) in einer Vielzahl von radialen Positionen entlang zumindest eines Abschnitts der Länge des röhrenförmigen Glieds vorgesehen sind, wobei jeweils positionierte zweite Verknüpfungen (68) vorgesehen sind, um dem allgemein röhrenförmigen Glied eine Krümmung in einer Vielzahl von Ebenen zu verleihen.

7. Intraluminale Vorrichtung (100) nach einem der vorhergehenden Ansprüche mit zumindest zwei ersten Verknüpfungen (31) unterschiedlicher Längen relativ zueinander; und/oder mit zumindest zwei zweiten Verknüpfungen (68) unterschiedlicher Längen relativ zueinander.

8. Intraluminale Vorrichtung (100) nach einem der vorhergehenden Ansprüche, worin die ersten Verknüpfungen (31) und/oder die zweiten Verknüpfungen (68) eine variable Längsabmessung aufweisen, worin optional die variable Längsabmessung durch eine Biegung oder Kurve in der Verknüpfung bereitgestellt wird.

9. Intraluminale Vorrichtung (100) nach einem der vorhergehenden Ansprüche, worin ein oder mehrere der strukturellen Elemente (30, 35), der ersten Verknüpfungen (31) und der zweiten Verknüpfungen (68) aus einer superelastischen Legierung geformt sind, worin optional die superelastische Legierung Nitinol ist; und/oder worin die Vorrichtung zumindest eine röntgenopake Markierung (71, 72) aufweist, die auf die radiale Orientierung der Vorrichtung hinweist; und/oder worin die Vorrichtung zumindest eine der folgenden Komponenten aufweist: einen Stent, eine Stent-Graftprothese, einen Filter und eine Verschlussvorrichtung.

10. Intraluminale Vorrichtung (100) nach einem der vorhergehenden Ansprüche, worin das allgemein röhrenförmige Glied im komprimierten Zustand eine im Wesentlichen zylindrische Konfiguration annimmt.

11. Intraluminale Vorrichtung (100) nach einem der vorhergehenden Ansprüche, worin bei Kompression der Vorrichtung die Gesamtlängen des Abschnitts des Glieds, der die Reihe von ersten Verknüpfungen (31) aufweist, und des Abschnitts des Glieds, der die Reihe von zweiten Verknüpfungen (68) aufweist, im Wesentlichen gleich sind, um der intraluminalen Vorrichtung eine im Wesentlichen gerade Gesamtgestalt zu verleihen.

12. Intraluminale Vorrichtung (100) nach einem der vorhergehenden Ansprüche, worin die strukturellen Elemente (30, 35) ohne Versatz ausgerichtet sind, worin optional benachbarte strukturelle Elemente (30, 35) so konfiguriert sind, dass sie als Spiegelbilder voneinander erscheinen.

13. Ablagevorrichtung mit einem Katheter (900), einer intraluminalen Vorrichtung (100) nach einem der vorhergehenden Ansprüche und einer Schleuse (904), welche die intraluminale Vorrichtung bedeckt; worin sich die intraluminale Vorrichtung in einer komprimierten Konfiguration auf dem Katheter befindet und im Wesentlichen gerade ist.

## Revendications

1. Dispositif intraluminal (100), comprenant :
une série d'éléments structuraux (30, 35, 34, 37) s'étendant dans une direction globalement longitudinale pour former un organe globalement tubulaire et comportant des sections disposées pour fournir une série de pics (38, 50, 66, 80, 82, 84, 86) et de creux (70, 71, 83, 85, 87), lesdits éléments structuraux étant reliés entre eux par au moins une série de premières liaisons (31, 202) et au moins une série de deuxièmes liaisons (68, 205), dans lequel lesdites premières liaisons sont plus courtes en longueur que lesdites deuxièmes liaisons, lesdites premières et lesdites deuxièmes liaisons étant disposées radialement et longitudinalement le long dudit organe globalement tubulaire,
le dispositif étant **caractérisé en ce qu'**une première série d'éléments structuraux (30, 35) est contenue dans une première zone (40) s'étendant le long d'un premier côté (98) de l'organe avec les pics (66) d'un élément structurel (30) étant reliés aux pics (50) d'un élément structurel adjacent (35) par les premières liaisons, **en ce qu'**une deuxième série d'éléments structuraux (34, 37) est contenue dans une deuxième zone (51) s'étendant le long d'un deuxième côté (99) de l'organe avec les creux (83) d'un élément structurel (34) étant reliés aux creux (87) d'un élément structurel adjacent (37) par les deuxièmes liaisons, et **en ce que** l'agencement et les longueurs des liaisons et des organes d'entretoises sont tels que, lorsqu'il est comprimé radialement, l'organe globalement tubulaire est obligé d'adopter une forme sensiblement droite et, lorsqu'il déployé radialement et dans un état détendu, la première zone (40) a une longueur totale (L₁) plus courte que la longueur totale (L₂) de la deuxième zone (51) de façon à donner audit organe globalement tubulaire une forme courbe.

2. Dispositif intraluminal (100) selon la revendication 1, dans lequel le dispositif intraluminal comprend un stent destiné à être déployé dans la partie de l'artère fémorale superficielle recouvrant le genou.

3. Dispositif intraluminal (100) selon l'une quelconque des revendications précédentes, dans lequel lesdites premières liaisons (202) et lesdites deuxièmes liaisons (205) comportent des parties flexibles pouvant fléchir lorsque ledit organe tubulaire est dans une configuration globalement droite et pouvant au moins partiellement retrouver sa forme droite lorsque ledit organe tubulaire est dans ladite configuration globalement courbe.

4. Dispositif intraluminal (100) selon l'une quelconque des revendications précédentes, dans lequel chacune des premières liaisons (303) est sensiblement inextensible longitudinalement et chacune des deuxièmes liaisons (302) est flexible de façon à être extensible longitudinalement ; dans lequel chaque deuxième liaison, lorsque en extension, a une longueur dans le sens longitudinal qui est au moins 1% plus grande que les longueurs des premières liaisons.

5. Dispositif intraluminal (100) selon l'une quelconque des revendications précédentes, dans lequel les premières liaisons (31) s'étendent le long d'une position radiale commune sur la longueur de l'organe globalement tubulaire et les deuxièmes liaisons (68) s'étendent le long d'une position radiale opposée le long de l'organe globalement tubulaire ; courbant ainsi l'organe globalement tubulaire dans un seul plan.

6. Dispositif intraluminal selon l'une quelconque des revendications 1 à 5, dans lequel les premières liaisons (31) sont fournies dans une pluralité de positions radiales le long d'au moins une partie de la longueur de l'organe tubulaire, avec les deuxièmes liaisons (68) positionnées respectivement, pour donner ainsi à l'organe globalement tubulaire une courbure dans une pluralité de plans.

7. Dispositif intraluminal (100) selon l'une quelconque des revendications précédentes, comprenant au moins deux premières liaisons (31) de longueurs différentes l'une par rapport à l'autre ; et/ou comprenant au moins deux deuxièmes liaisons (68) de longueurs différentes l'une par rapport à l'autre.

8. Dispositif intraluminal (100) selon l'une quelconque des revendications précédentes, dans lequel les premières liaisons (31) et/ou les deuxièmes liaisons (68) ont une dimension longitudinale variable, ladite dimension longitudinale variable étant éventuellement fournie par une flexion ou une courbe dans le liaison.

9. Dispositif intraluminal (100) selon l'une quelconque des revendications précédentes,
dans lequel un ou plusieurs éléments structuraux (30, 35), les premières liaisons (31) et les deuxièmes liaisons (68) sont formés dans un alliage super-élastique, l'alliage super-élastique étant éventuellement du nitinol ; et/ou
dans lequel le dispositif comprend au moins un marqueur (71, 72) radio-opaque indiquant l'orientation radiale du dispositif ; et/ou
dans lequel le dispositif comprend au moins un des éléments suivants : un stent, un greffon de stent, un filtre et un dispositif d'occlusion.

10. Dispositif intraluminal (100) selon l'une quelconque des revendications précédentes, dans lequel l'organe globalement tubulaire adopte une configuration sensiblement cylindrique lorsqu'il est dans un état comprimé.

11. Dispositif intraluminal (100) selon l'une quelconque des revendications précédentes, dans lequel lorsque le dispositif est comprimé les longueurs totales de la partie de l'organe comprenant la série des premières liaisons (31) et la partie de l'organe comprenant la série des deuxièmes liaisons (68) sont sensiblement les mêmes, de façon à donner au dispositif intraluminal une forme globale qui est sensiblement droite.

12. Dispositif intraluminal (100) selon l'une quelconque des revendications précédentes, dans lequel les éléments structuraux (30, 35) sont alignés sans décalage, les éléments structuraux adjacents (30, 35) étant éventuellement configurés pour apparaître comme des images symétriques les uns des autres.

13. Dispositif de mise en place comprenant un cathéter (900), un dispositif intraluminal (100) selon l'une quelconque des revendications précédentes et une gaine (904) recouvrant le dispositif intraluminal ; dans lequel le dispositif intraluminal est dans une configuration comprimée sur le cathéter et sensiblement droit.
